# EUROPEAN PATENT APPLICATION

(11) **EP 1 865 069 A1**
(43) Date of publication of application: **12.12.2007**
(21) Application number: 05815943.5
(22) Date of filing: 15.11.2005
(51) Int. Cl.: C12Q 1/68

(54) **DNA ANALYSIS-BASED METHOD FOR THE IDENTIFICATION OF SPECIES OF PARTRIDGE AND THE DETECTION OF HYBRIDS OF THE GENUS ALECTORIS**

(30) Priority: 18.03.2005 ES 200500715
(71) Applicant: Universidad de Zaragoza, E-50005 Zaragoza (ES)
(72) Inventor: ARRUGA LAVIÑA, María Victoria, E-50005 Zaragoza (ES); GARCÍA GARCÍA, Cristina Belén, E-50005 Zaragoza (ES); MONTEAGUDO IBÁÑEZ, Luis Vicente, E-50005 Zaragoza (ES)
(74) Representative: Pons Arino, Angel
(86) International application number: PCT/ES2005/000621
(87) International publication number: WO 2006/097549

(57) **Abstract**

The invention relates to a DNA analysis -based method for the identification of different species of the genus *Alectoris* and the hybrids thereof. Said analysis s performed (i) using two polymorphisms which are located at position 234 of the mitochondrial cytochrome b gene and at position - 526 of intron 1 of the rhodopsin gene respectively and (ii) studying of RAPD-type patterns. In addition, the invention also relates to a diagnostic kit which is used to perform said analysis, together with the necessary primers or initiators.

## Description

### FIELD OF THE INVENTION

The present invention refers to the identification of different species of the *Alectoris* genus and detection of hybrids based on DNA analysis. More specifically, the invention consists of a procedure for identifying the species and detecting hybrids of said genus, by the use of single nucleotide polymorphisms (SNP) and RAPD patterns.

### BACKGROUND OF THE INVENTION

Seven different species of partridge are currently known that make up the *Alectoris* genus. The red-legged partridge *(Alectoris rufa)* is the only native Spanish partridge apart from small areas where the Barbary partridge *(Alectoris barbara)* is still found in the South of the country.

In the case of the red-legged partridge, when it is crossed with other partridges of other species hybrids are obtained that are easily identifiable by their outer morphology. The problem arises when after successive crossings of a red-legged partridge hybrid with a pure specimen of red-legged partridge the outer morphological characteristics of the hybrid become diluted such that a hybrid red-legged partridge is finally obtained that cannot be distinguished from a pure red-legged partridge.

This dilution of the outer morphological features is especially problematic in hybrids obtained from crossing *A. rufa* with *A. graeca* or with *A. chukar,* since other hybrids show features in their plumage that make them easily distinguishable with the naked eye, even after successive crossings with pure specimens of *A. rufa.*

The Law 4/1989 of March 27 on the Conservation of Natural Spaces and Wild Flora and Fauna prohibits the release of foreign species and their hybrids in Spanish territories. It is therefore of productive and ecological interest to develop systems that allow generally differentiating native species from foreign species and their hybrids, especially native species *Alectoris rufa* from other species of the *Alectoris* genus *(A. arabian, A. melanocephala, A. chukar, A. magna, A. graeca, A. philbyi* and *A. barbara)* and *A. rufa* hybrids.

Several procedures based on protein analysis have been developed for differentiating species of the *Alectoris* genus (Randi et al., 1992, The Auk 111 (2): 416-426).

Studies of genetic material are another alternative that is commonly used in recent years. Amplified DNA, for example by means of polymerase chain reaction or PCR (Mullis and Faloona, 1987. Meth Enzymol 155: 335-350) can be used to generate DNA band patterns by electrophoresis in agarose or polyacrylamide gels. These band patterns are used in order to characterize or differentiate organisms using different initiators or primers, designed based on different DNA regions.

Usually, the initiators used originate band patterns that are different for each species. These initiators are used in techniques based on repeated sequences of DNA that are generically called rep-PCR and in techniques based on random DNA amplification called RAPD (Random Amplified Polymorphic DNA). (Cortes et al., 2001, Cortés, O.; Cañón, J and Dunner, S. 2001. Utilización de "pools" de ADN y RAPD para identificar diferencias genómicas entre la Perdiz Roja *(Alectoris rufa)* y Perdiz Griega (Alectoris graeca) (Use of DNA pools and RAPD to identify genome differences between Red-Legged Partridge (Alectoris rufa) and Greek Partridge (Alectoris graeca)). III Congreso de la Sociedad Española de Genética (III Congress of the Spanish Genetics Society). Seville 2001. p. 89.

Negro et al. (Negro, J.J., M.J. Torres & J.A. Godoy 2001. RAPD analysis for detection and eradication of hybrid partridges (Alectoris rufa x A. graeca) in Spain. Biological Conservation 98:19-24) develop a procedure based on a RAPD type analysis, starting from the analysis of a small number of supposedly pure partridge specimens, to identify the *A*. *rufa, A. graeca* species and hybrids thereof, which is unreliable since it only provides a single piece of evidence, a single marker. This procedure cannot therefore provide conclusive results, therefore it could be used as a complementary identification procedure but not as a definitive procedure, and in the case of Cortés et al. the study was equally performed with a single sample along with a pool or group of animals, which does not allow identification with respect to whether a single specimen belongs to the desired species or not, or whether it is a hybrid not.

Another technique based on DNA analysis, used in population studies, consists of the study of single nucleotide polymorphisms (SNP), which are stable mutations that are very useful as genetic markers. These can be identified by means of PCR amplification of known gene fragments, sequencing and comparing or by the use of computing tools that allow identifying a large number of candidate SNPs that must be subsequently validated by means of sequencing and identification in population samples (Buetow et al., 1999).

Validation is the critical phase of species identification and differentiation procedures by SNPs, since in most cases the SNPs found will not be useful for identifying and differentiating species.

Once the SNP has been validated a procedure must be prepared for fast and reliable detection. These methods usually consist in PCR amplification of the DNA fragment or fragments (amplicon) containing the SNP and identification by sequencing, SSCP (Secondary Structural Content Prediction), amplicon restriction, in the case that the polymorphism changes the recognition sequence for a restriction enzyme or by allelic discrimination by means of the RT-PCR (Real Time-PCR) procedure.

All that is mentioned above shows the need for developing a reliable procedure for the detection and identification of hybrid partridges.

### DESCRIPTION OF THE INVENTION

The present invention is the result of the search for new polymorphisms and RAPD patterns that allow identifying species of the *Alectoris* genus, as well as hybrids thereof. Two new single nucleotide polymorphisms (SNP) have been found, and an initiator or primer that allows obtaining RAPD patterns that allow said identification.

This invention provides a procedure, compounds and analysis kits for identifying and differentiating *A*. *rufa* specimens from other species of the *Alectoris* genus, as well as the hybrids obtained from crossing the former with *A. graeca* or *A*. *chuker* by means of nuclear and mitochondrial DNA analysis.

A first aspect of the present invention provides an analysis procedure for the identification and differentiation of different species of the *Alectoris* genus, as well as hybrid partridges of the *A. rufa* species the maternal line of which does not belong to said species, comprising detecting the presence in a biological sample (tissue or fluid) from a single specimen of a Thymine for Cytosine base change polymorphism (SNP1) in the -234 position of SEQ ID NO:1, located in the mitochondrial cytochrome b gene.

Specifically, the analysis procedure implies examining a partridge specimen by correlating it with the presence or absence of the polymorphism (i.e. the presence or absence of the most frequent allele). Said analysis uses the polymerase chain reaction (PCR), the single strand conformational polymorphism test (SSCP) or any other type of analysis that allows identifying the polymorphism. Thus, with the previously mentioned SNP it can be determined whether or not a specimen of red-legged partridge is a hybrid.

The analysis procedure further comprises the use of indicator means that react in the presence of one of the polymorphisms. The indicator means typically induce the production of a detectable signal in the presence of the polymorphism and can induce a change in colour or clotting or a restriction site, detectable by additional analytical steps. The initiator means can also comprise an antibody with binding affinity between the wild-type sequence and the polymorphism.

In one embodiment the analysis procedure comprises detecting the presence of a base change polymorphism, Thymine for Cytosine, in position - 234 of the mitochondrial cytochrome b gene. In a preferred embodiment, this detection takes place using PCR primers that amplify a DNA segment containing the nucleotide at position -234 of the mitochondrial cytochrome b gene. A more preferred embodiment uses a pair of PCR primers with sequences SEQ ID No:2 and SEQ ID No:3.

In another embodiment, the analysis procedure comprises a pair of PCR primers that amplify a mitochondrial DNA segment from the cytochrome b gene, in which the amplified DNA segment is approximately 800 bps in length, preferably up to 600 bps and more preferably up to 506 bps. In a preferred embodiment of this embodiment, the PCR primers would be those corresponding to sequences SEQ ID No:2 and SEQ ID No:3.

A second aspect of the invention provides another step to the analysis procedure comprising detection of a DNA fragment of between 1000 and 1100 bps in RAPD type patterns obtained by the use of a primer. A preferred embodiment uses a PCR primer with a sequence corresponding to SEQ ID No:4 or derived analogues. Thus, a specimen of red-legged partridge can be determined as pure or hybrid *(A. rufa-A. graeca* and *A. rufa-A. chukar)* given the presence or absence of said diagnostic fragment or band in the RAPD pattern.

This other step of the analysis procedure also comprises the joint detection of the diagnostic band described above and a second band (560 bps) in RAPD type patterns obtained by the use of a primer. A preferred embodiment uses a PCR primer with the sequence corresponding to SEQ ID No:4 or derived analogues. Thus, the presence of said pair of bands indicates that the sample comes from a pure specimen of Greek partridge.

In a particular embodiment, separation and visualization of the fragments or bands forming the RAPD patterns can be performed by procedures that are well-known in the state of the art, although in a preferred embodiment the amplification products are separated by means of standard submarine electrophoresis in agarose gel or, in general, in any matrix to which an electrical current can be applied that will allow molecule separation, and that can be visualized by means of ultraviolet light (W) after staining with ethidium bromide.

A third aspect of the invention provides another step to the analysis procedure for identifying and differentiating hybrid partridges, comprising detecting in a biological sample (tissue or fluid) from a single specimen the presence of a second Thymine for Cytosine base change polymorphism (SNP2) at position -526 of intron 1 of the rodopsin gene (RDP1) corresponding with position -49 of the sequence corresponding to SEQ ID No:5, accessible from the Genbank database with access key AF394641. In said sequence (AF394641) the SNP2 is at position 533.

Specifically, the analysis procedure implies examining a partridge specimen by correlating it with the presence or absence of the polymorphism (i.e. the presence or absence of the most frequent allele). Said analysis can use the polymerase chain reaction (PCR), the single strand conformational polymorphism (SSCP) or any other type of analysis that allows identifying the polymorphism. Thus, and given the SNP2, it can be determined whether a red-legged partridge specimen is pure or hybrid. Additionally, this third aspect of the invention also provides a test to determine whether a sample belongs to a pure specimen of *A. chukar.*

In one embodiment, the analysis procedure comprises detecting the presence of a Thymine for Cytosine base change polymorphism (SNP2) in position -526 of intron 1 of the rodopsin gene. In a preferred embodiment, detection is carried out using PCR primers that amplify a DNA fragment containing the nucleotide at position -526 of intron 1 of the rodopsin gene. A more preferred embodiment uses a pair of PCR primers with sequences SEQ ID No:6 and SEQ ID No:7.

In another embodiment, the analysis procedure comprises a pair of PCR primers that amplify a segment of the rodopsin gene, in which the amplified DNA segment is of approximately 800 bps in length, preferably up to 600 bps, and more preferably up to 441 bps. In a preferred embodiment of this embodiment the PCR primers would be those corresponding to sequences SEQ ID No:6 and SEQ ID No:7.

The invention also provides diagnostic kits for analyzing and detecting pure and hybrid partridges of the *A. rufa* species, comprising diagnostic means according to the first, second and third aspect of the invention, optionally in a pack. In a particular embodiment the analysis kit comprises a transport means such as a compartmentalized box housing the analyzing means according to the invention, optionally within a pack such as a flask, a tube, a blister or similar.

Along the entire description and the claims of the specification, the word "comprises" and variations thereof, such as "comprising" do not intend to exclude other additives, components, members or steps. Both the embodiment and the enclosed drawings do not intend to limit the invention, but should rather be taken as an aid towards better understanding the invention.

### DESCRIPTION OF THE DRAWINGS

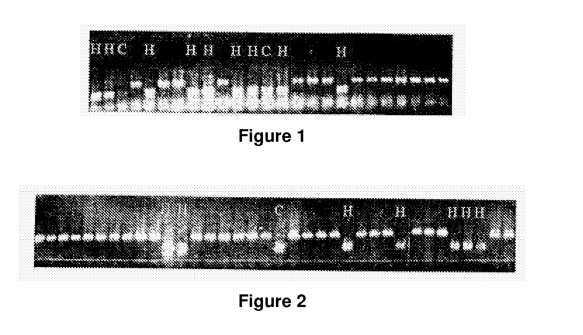

**Figure 1** shows a 1% agarose gel in which the C lanes correspond to lanes loaded with a DNA sample from pure specimens of *A. chukar* and the H lanes correspond to lanes loaded with a DNA sample from *A. rufa* hybrids, the maternal line of which belongs to the *A*. *chukar* species. Both samples show the presence of two bands (271 and 235 bps) from DNA digestion; the remaining lanes correspond to samples from pure specimens of *A*. *rufa* or partridge hybrids whose maternal line belongs to the *A. rufa* species, with a 506 bp band.
**Figure 2** shows a 2% agarose gel in which the C lanes correspond to lanes loaded with a DNA sample from pure specimens of *A. chukar* and the **H** lanes correspond to lanes loaded with a DNA sample from *A. rufa* hybrids, the maternal line of which belongs to the *A. chukar* species. Both samples show the presence of two bands (271 and 235 bps) from DNA digestion; the remaining lanes correspond to samples from pure specimens of *A*. *rufa* or partridge hybrids whose maternal line belongs to the *A. rufa* species, with a 506 bp band.

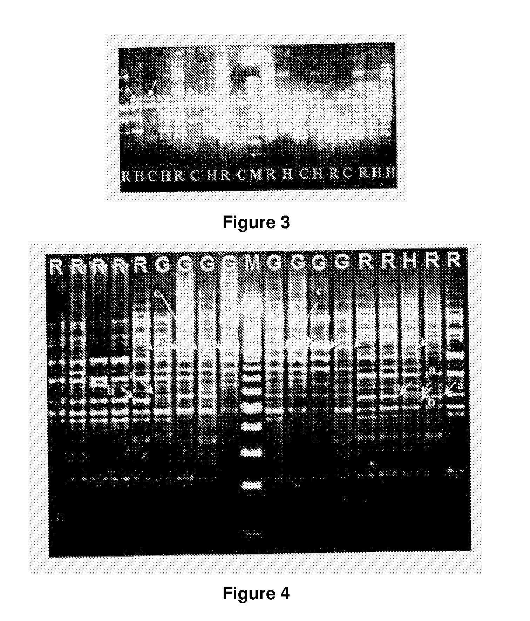

**Figure 3** shows an agarose gel in which the C lanes correspond to lanes loaded with a DNA sample from pure specimens of *A. chukar;* the **H** lanes correspond to lanes loaded with a DNA sample from hybrid partridges *(A. rufa-A. chukar).* Both lanes show the presence of a diagnostic band (1000-1100 bps), indicated with an arrow, that allow identification; the R lanes correspond to lanes loaded with a sample from pure specimens of *A*. *rufa.* The **M** lane corresponds to the lane with the molecular weight markers.
**Figure 4** shows an agarose gel in which the **H** lanes correspond to lanes loaded with a DNA sample from hybrid partridges *(A. rufa-A. chukar);* the **G** lanes correspond to lanes loaded with a DNA sample from pure specimens of *A. chukar.* Both lanes show the presence of a diagnostic band from 1000 to 1100 bps **(c arrows)** and the G lanes also show a second diagnostic band from **500** to **600 bps (a arrows);** the R lanes correspond to lanes loaded with a sample from pure specimens of *A*. *rufa.* The **M** lane corresponds to the lane with the molecular weight markers.

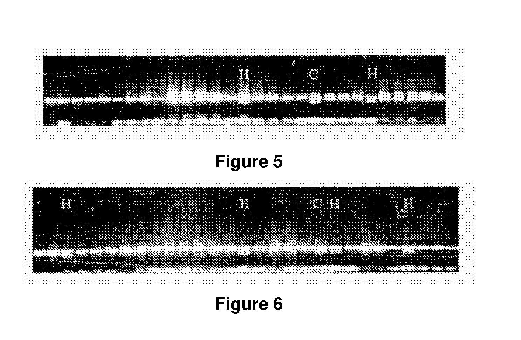

**Figure 5** shows an agarose gel in which the **C** lanes correspond to lanes loaded with a DNA sample from pure specimens of *A. chukar;* the **H** lanes correspond to lanes loaded with a DNA sample from hybrid *A*. *rufa* partridges. Both lanes show the presence of two bands (354 and 87 bps) from DNA digestion; the remaining lanes correspond to samples from pure specimens of *A. rufa* (441 bps).
**Figure 6** shows an agarose gel in which the C lanes correspond to lanes loaded with a DNA sample from pure specimens of *A. chukar;* the **H** lanes correspond to lanes loaded with a DNA sample from hybrid *A. rufa* partridges. Both lanes show the presence of two bands (354 and 87 bps) from DNA digestion; the remaining lanes correspond to samples from pure specimens of *A. rufa* (441 bp band).

### EMBODIMENT

The following experimental test illustrates the invention carried out and reveals the specificity and effectiveness of the procedure.

### 1. Obtaining specific restriction patterns that allow the differentiation of pure and hybrid specimens of A. rufa and other species of the Alectoris genus

This test was performed in order to obtain restriction patterns that would allow identifying pure and hybrid *A*. *rufa* specimens.

### 1.1. Search for candidate SNPs that will allow identifying different species of the Alectoris genus, of A. rufa and hybrids thereof

Comparison of gene sequences obtained from public databases (GenBank database) and their analysis by means of the BIOEDIT programme led to detecting a possible SNP candidate (SNP1), present in mitochondrial DNA, more specifically in the cytochrome B gene. Said SNP1 was different in the *A*. *chukar, A. graeca* and *A*. *rufa* species.

### 1.2. SNP validation

Different *A. rufa-A. chukar,* hybrids were tested, which were compared to pure specimens of *A. rufa* and other species of the *Alectoris* genus.

### 1.3. Method

DNA extractions from the specimens to be analyzed were performed from blood in tubes with EDTA, blood samples deposited in FTA® cards, feathers or any other biological tissue, following the extraction from blood protocol below:
1) Place 50 microliters of blood and 1.3 milliliters of sterile double-distilled water in an Eppendorf tube (1.5 milliliters).
2) Incubate for 30 minutes at room temperature. Shake twice so it is well homogenized.
3) Centrifuge for 2 minutes and discard the supernatant.
4) Add 200 microliters of 5% Chelex to the pellet.
5) Transfer all the material to a 500 microliter Eppendorf tube and vortex.
6) Incubate at 56ºC for 15 minutes.
7) Vortex and incubate at 100ºC for 15 minutes.
8) Preserve at -20ºC.

PCR for DNA amplification from the samples was performed using 50 to 100 nanograms of DNA, two primers with sequences SEQ ID No:2 and SEQ ID No:3, respectively along with the necessary reagents.

PCR conditions were the following: preheating at 94ºC for 3 minutes; 35 denaturing cycles at 94ºC for 30 seconds; annealing at 60ºC for 1 minute; and elongation at 72ºC for 30 seconds. PCR products were kept at 4ºC and at -20ºC when the following step of the analysis was delayed for more than two hours.

Having performed the PCR, 8.5 microliters of PCR product were subjected to the enzymatic action of the BsaAl restriction enzyme (0.5 microliters of the enzyme and 1 microliter of 10X Buffer recommended by the manufacturer), or one of its isoschizomers, capable of recognizing the presence of the candidate SNP that had been detected at the beginning of the present example. Digestion was performed at 37ºC for 4 hours in a water bath.

Having finished digestion, a standard submarine electrophoresis was performed with the products of said digestion, in an agarose gel at a concentration of 1% in TBEx1 (tris, boric acid, EDTA) at a voltage of 40 volts for 10 minutes and 100 volts for 30 minutes, and they were stained with ethidium bromide at a concentration of 0.5 micrograms per milliliter of TBE1X. The restriction patterns were visualized and photographed when the agarose gel was subjected to ultraviolet light through a transilluminator after electrophoresis. 1.4. Results

The results showed that two different restriction patterns were obtained according to the origin of the samples tested.

A restriction pattern was obtained with a single band corresponding to the amplified DNA fragment (506 base pairs) that had not been cut by the restriction enzyme in the case of hybrid *A. rufa* specimens whose maternal line belonged to the *A. rufa* species and in the case of pure *A. rufa* specimens. And a second restriction pattern was obtained with two bands of 271 and 235 base pairs, respectively (**Figures 1 and 2, lanes H and C**), corresponding to the two DNA fragments originating from the digestion carried out by the restriction enzyme, in the case of hybrid specimens whose maternal line did not belong to the *A*. *rufa* species as well as for those specimens belonging to other species of the *Alectoris* genus in purity.

### 1.5. Interpretation of the results.

This first step of the identification and differentiation procedure provides, according to the results obtained, two restriction patterns that allow identifying those hybrid specimens whose maternal line does not belong to the *A*. *rufa* species.

Pure hybrids of *A*. *rufa* whose maternal line belongs to the *A*. *rufa* species, and pure specimens of *A*. *rufa* cannot be differentiated with this first test, therefore it is necessary to continue with the identification and differentiation procedure.

### 2. Obtaining specific RAPD patterns that allow identification of pure specimens of A. rufa and hybrids of A. rufa with other species of the Alectoris genus

This test was performed in order to obtain RAPD patterns that would allow differentiating and identifying pure specimens and hybrids of *A*. *rufa.*

### 2.1. Procedure

DNA extractions were performed from partridge specimens that had been identified in the first step of the identification and differentiation procedure as individuals belonging to the *A*. *rufa* species from samples following the same extraction protocol as in the first step of the procedure.

PCR for DNA amplification from the samples was performed using 2.5 microliters of DNA, a single primer with SEQ ID No:4 or derived analogues, along with all the necessary reagents.

PCR conditions were the following: preheating at 94ºC for 4 minutes; 45 denaturing cycles at 94ºC for 1 minute; annealing at 36ºC for 1 minute; elongation at 72ºC for 2 minutes; and a final elongation at 72ºC for 10 minutes. PCR products were kept at 4ºC and at -20ºC when the following step of the analysis was delayed for more than two hours.

Having performed the PCR, the entire product obtained was loaded in an agarose gel at a concentration of 1.5% in TBEx1 (tris, boric acid, EDTA) at a voltage of 40 volts for 10 minutes, 95 volts for 30 minutes and 120 volts for 45 minutes, and they were stained with ethidium bromide at a concentration of 0.5 micrograms per milliliter of TBE1X.

Having finished the electrophoresis, the RAPD patterns were visualized and photographed after illuminating the agarose gel with ultraviolet light through a transilluminator.

### 2.2. Results

The results obtained after testing multiple hybrid specimens with different primers showed the irreproducibility of these RAPD patterns that varied significantly between hybrid specimens and did not allow their differentiation with respect to pure specimens.

Repetition of this test with multiple primers led to discovering a primer that allowed differentiating between hybrid, pure *A. graeca* and pure *A rufa* specimens. This primer gave rise to a RAPD band pattern (**Figures 3 and 4**) that although different between the different hybrid samples tested, showed a common feature, defined by the presence of a diagnostic band (**Figures 3 and 4, c arrows**) between 1000 and 1100 bps. Additionally, the presence of another smaller diagnostic band (500 to 600 bps) was detected (**Figures 3 and 4, a arrows**).

### 2.3. Interpretation of the results

This second step of the identification and differentiation procedure provides, according to the results obtained, a series of RAPD patterns that allow differentiation of pure specimens of *A*. *rufa* with respect to *A*. *rufa* hybrids, not identifiable by means of the first identification procedure, and other species of the *Alectoris* genus.

The presence of the aforementioned 1000 to 1100 bp diagnostic band in RAPD patterns obtained by using a single primer, provided by the present invention, or derived analogues, indicates that the samples tested come from a hybrid specimen of red-legged partridge (*A*. *rufa-A. graeca* or *A*. *rufa-A. chukar*)*.* Furthermore, the joint presence of said diagnostic band together with a second band of between 500 and 600 bps indicates that the sample comes from a pure specimen of *A*. *graeca* (**Figure 4, a arrows**).

### 3. Obtaining specific restriction patterns that allow differentiation and identification of pure and hybrid specimens of A. rufa.

This test was performed with the aim of obtaining restriction patterns that would allow identifying hybrid *A. rufa* specimens obtained from crossings with specimens belonging to the *A. chukar* or *A. graeca* species, such that a third test was obtained that would verify the results obtained in the second identification step of the present invention.

### 3.1. Search for candidate SNPs that will allow identifying pure and hybrid specimens of A. rufa

Comparison of gene sequences obtained by direct sequencing of PCR products and their analysis by means of the BIOEDIT programme led to detecting a possible SNP candidate (SNP2), present in chromosomal DNA, more specifically in the RDP1 or rodopsin gene. Said SNP2 is different in the specimens of the *A. rufa* hybrids mentioned and pure specimens of *A. rufa.*

### 3.2. SNP validation

Different *A. rufa* hybrids were tested, which were compared to pure specimens of *A. rufa* and other species of the *Alectoris* genus.

### 3.3. Procedure

### 1) DNA extractions from the specimens to be analyzed were performed from biological samples following the same protocol used in the two previous steps of the identification procedure.

PCR for DNA amplification from the samples was performed using 1.6 microliters of DNA, two primers with sequences SEQ ID No:6 and SEQ ID No:7, respectively, along with the necessary reagents.

PCR conditions were the following: preheating at 94ºC for 4 minutes and 30 seconds; 35 denaturing cycles at 94°C for 30 seconds; annealing at 64ºC for 45 seconds; and elongation at 72ºC for 1 minute; finishing with a final step at 72ºC for 7 minutes. PCR products were kept at 4ºC and at -20ºC when the following step of the analysis was delayed for more than two hours.

Having performed the PCR, 8 microliters of PCR product were subjected to the enzymatic action of the Apal enzyme (0.5 microliters of the enzyme and 0.5 microliters of I Buffer recommended by the manufacturer), or one of its isoschizomers, capable of recognizing the presence of the candidate SNP2 that had been detected at the beginning of the present example. Digestion was performed at 37ºC for 4 hours in a water bath.

Having finished digestion, a standard submarine electrophoresis was performed with the products of said digestion, in an agarose gel at a concentration of 1.5 percent in TBEx1 (tris, boric acid, EDTA) at a voltage of 40 volts for 10 minutes and 100 volts for 30 minutes, and they were stained with ethidium bromide at a concentration of 0.5 micrograms per milliliter of TBE1X. The restriction patterns were visualized and photographed when the agarose gel was subjected to ultraviolet light through a transilluminator after electrophoresis.

### 3.4. Results

The results showed that two different restriction patterns were obtained according to the origin of the samples tested.

A restriction pattern was obtained with a single band corresponding to the amplified DNA fragment (411 base pairs) that had not been digested by the restriction enzyme in the case of pure *A. rufa* specimens. And a second restriction pattern was obtained with two bands of 354 and 87 base pairs, respectively (**Figures 5 and 6**), corresponding to the two DNA fragments originating from the digestion carried out by the restriction enzyme, in the case of hybrid specimens of *A. rufa* and pure specimens of *A. chukar.*

### 3.5. Interpretation of the results

This third step of the identification and differentiation procedure provides, according to the results obtained, two restriction patterns that allow: a) identifying those hybrid *A*. *rufa* specimens that had not been detected by the two previous steps of the identification method described in the present embodiment; b) verifying or confirming the purity of the *A. rufa* specimens detected as such by the previous two steps of the identification procedure described in the present embodiment.

## Claims

1. An identification procedure for identifying different species of the *Alectoris* genus and hybrids thereof based on DNA analysis, **characterized in that** it comprises detection of the presence or absence of (i) two polymorphisms located at the -234 position of the cytochrome b mitochondrial gene and in position -526 of intron 1 of the rodopsin gene, respectively, and (ii) of several diagnostic bands in RAPD type patterns from a tissue or fluid sample from a single specimen.

2. A procedure according to claim 1, **characterized in that** one of the polymorphisms is a change of Thymine for Cytosine in position -234, in the cytochrome b mitochondrial gene.

3. A procedure according to claim 2, **characterized in that** the polymorphism is detected using PCR primers that amplify a fragment of mitochondrial DNA, which comprises the nucleotide in position -234 of the cytochrome b mitochondrial gene.

4. A procedure according to claim 3, **characterized in that** the PCR primers have sequences SEQ ID No:2 and SEQ ID No:3.

5. A procedure according to claim 1, **characterized in that** one of the polymorphisms consists in a change of Thymine for Cytosine in position -526 of intron 1 of the rodopsin gene.

6. A procedure according to claim 5, **characterized in that** the polymorphism is detected using PCR primers that amplify a fragment of DNA comprising the nucleotide in position -526 of intron 1 of the rodopsin gene.

7. A procedure according to claim 6, **characterized in that** the PCR primers have sequences SEQ ID No:6 and SEQ ID No:7.

8. A procedure according to claim 1, **characterized in that** one of the diagnostic bands corresponds to a DNA fragment of between 1000 and 1100 bps.

9. A procedure according to claim 1, **characterized in that** another of the diagnostic bands corresponds to a DNA fragment of between 500 and 600 bps.

10. A procedure according to claim 8, **characterized in that** the RAPD type patterns are obtained by the use of a primer with sequence SEQ ID No:4 or derived analogues.

11. Isolated DNA **characterized in that** it comprises a region of mitochondrial cytochrome b gene in which a Thymine in position -234 has been substituted by a Cytosine.

12. Isolated DNA according to claim 10, **characterized in that** it comprises sequence SEQ ID No:1.

13. Isolated DNA **characterized in that** it comprises a region of intron 1 of the rodopsin gene in which a Thymine in position -526 has been substituted by a Cytosine.

14. DNA according to claim 12, **characterized in that** it comprises sequence SEQ ID No:5.

15. Isolated DNA of between 1000 and 1100 base pairs, **characterized in that** it is obtained by the use of a primer with sequence SEQ ID No:4.

16. A pair of PCR primers amplifying a DNA fragment of the cytochrome b gene, **characterized in that** the amplified DNA has a length of up to 800 bps with an SNP according to claim 2 located within it.

17. A primer chosen from the group formed by SEQ ID No:2 and SEQ ID No:3.

18. A PCR primer chosen from the group formed by SEQ ID No:4 and derived analogues.

19. A pair of PCR primers amplifying a DNA fragment of the rodopsin gene, **characterized in that** the amplified DNA has a length of up to 800 bps with an SNP according to claim 5 located within it.

20. A primer chosen from the group formed by SEQ ID No:6 and SEQ ID No:7.

21. An identification kit for hybrids of the *Alectoris* genus, **characterized in that** it comprises primers according to claims 15, 17 and 18.


SEQUENCE LIST
<110> Zaragoza University

<120> Procedure for identifying and differentiating hybrid partridges of the
Alectoris rufa species.

<130> Reference number

<160> 7

<170> PatentIn version 3.1

<210> 1
<211> 507
<212> DNA
<213> Alectoris rufa
<223> Sequence corresponding to the cytochrome B gene

<400> 1
cactacaccg cagacaccac cctcgccttc tcatcagtag cccacacatg tcgaaacgta 60
cagtacggct gactcatccg caacctccat gcaaacggcg cctcattctt tttcatctgc 120
atcttcctcc acatcggacg cggcctatac tatggctctt atctctacaa agaaacttga 180
aacacaggag taattctgct cctcacacta atagccactg ctttcgtagg ctatgtactc 240
ccatggggcc aaatatcctt ctgaggggct accgtcatca caaacctatt ctcagccgtc 300
ccctacatcg gacaaaccct agtagagtga gcctggggag gattctcagt cgacaaccca 360
accctcaccc gattcttcgc cctccacttc ctcctcccct tcgtaattgc aggaattacc 420
atcatccacc tcacattcct ccacgaatcc ggctcaaaca atccactagg catctcatca 480
aactccgaca aaatcccatt tcaccca 507

<210> 2
<211> 507
<212> DNA
<213> Artificial sequence

<223> Direct oligonucleotide initiator

<400> 2

cactacaccg cagacaccac 20

<210> 3

<211> 20

<212> DNA

<213> Artificial sequence

<223> Indirect oligonucleotide initiator

<400> 3
tgggtgaaat gggattttgt 20

<210> 4

<211> 10

<212> DNA10:49 08/03/2005

<213> Artificial sequence

<223> Direct oligonucleotide initiator

<400> 4
tgccgagctg 10

<210> 5

<211> 369

<212> DNA

<213> Alectoris rufa

<223> Sequence corresponding to the Rodopsin gene

<400> 5
cggctcagct cccagccata ctgcaggcag ctgttggcgg ggttgggctc acaaggcacg 60

gatggcctga gcagaacctg ggggcagtgc tgcagctcag agcggggcca tgggcacgtg 120

ggtgcagtgc tgtgtgggag gagggcaggt ccagtggtgg atctcagagc acagcagcgc 180

agcagttgcc gctggggatc tggaggcatc ttctgagtcc ttcccaccac actggtactc 240

gtcctcagtt ccattcagtt tctgagcacg gggtggaggt tcagtgctga taggggtctt 300

cctcttgcca gcaatggcat gcctttgtcc tctcgtcact tgaggtccct atatctccat 360

ccctttctc 369

<210> 6

<213> 20
<212> DNA
<213> Artificial sequence
<223> Direct oligonucleotide initiator

<400> 6
aaataagggc cgtgtgtctg 20

<210> 7
<211> 20
<212> DNA
<213> Artificial sequence
<223> Indirect oligonucleotide initiator

<400> 7
acgagtgacc agagagcgat 20